Europäisches Patentamt

European Patent Office

Office européen des brevets

(19)

(11) Numéro de publication : **0 306 377 B1**

# FASCICULE DE BREVET EUROPEEN

(12)

(45) Date de publication du fascicule du brevet :
**04.03.92 Bulletin 92/10**

(51) Int. Cl.⁵ : **A61K 33/30,** A61K 33/00,
// (A61K33/30, 33:14, 33:04,
33:00)

(21) Numéro de dépôt : **88402036.3**

(22) Date de dépôt : **04.08.88**

(54) **Nouvelles compositions d'éléments minéraux et/ou oligoéléments et leur procédé de préparation.**

(30) Priorité : **14.08.87 FR 8711579**

(43) Date de publication de la demande :
**08.03.89 Bulletin 89/10**

(45) Mention de la délivrance du brevet :
**04.03.92 Bulletin 92/10**

(84) Etats contractants désignés :
**AT BE CH DE ES FR GB GR IT LI LU NL SE**

(56) Documents cités :
**DICTIONNAIRE VIDAL, 1986, OVP, Paris, FR
ROTE LISTE, 1965, Editio Cantor, Aulen-
dorf/Württ**

(73) Titulaire : **Christophe, Claude
4 rue Waldteufel
F-67000 Strasbourg (FR)**

(72) Inventeur : **Leroy, Lise neé Punch
20 rue de Labaroche
F-67100 Strasbourg (FR)**
Inventeur : **Christophe, Claude
4 rue Waldteufel
F-67000 Strasbourg (FR)**

(74) Mandataire : **Brycman, Jean et al
CABINET ORES 6, Avenue de Messine
F-75008 Paris (FR)**

EP 0 306 377 B1

## Description

La présente invention est relative à de nouvelles compositions d'éléments minéraux et/ou oligoéléments, à un procédé pour les préparer et à leur utilisation en thérapeutique humaine et vétérinaire en dermato-cosmétologie et en diététique.

Il est connu qu'un certain nombre d'éléments minéraux et/ou oligoéléments dits essentiels sont indispensables à l'organisme vivant, tant humain qu'animal, en vue d'assurer un certain nombre de fonctions vitales dudit organisme, notamment en ce qui concerne les différents métabolismes.

L'absence ou l'insuffisance de ces éléments se traduit par des anomalies de croissance ou de développement ou des pathologies particulières, qui peuvent s'accompagner de symptômes spécifiques de carences.

Parmi les oligoéléments et éléments minéraux essentiels et indispensables à la vie, on peut citer, par exemple, notamment :

| . potassium | . iode |
| . calcium | . magnésium |
| . zinc | . sélénium |
| . cuivre | . silicium |
| . fer | . fluor |
| . cobalt | . brome |
| . nickel | . soufre |
| . chrome | . phosphore |
| . vanadium | . lithium |
| . molybdène | . germanium |
| . manganèse | . sodium |
| . rubidium | . étain |

Ils sont impliqués dans de très nombreuses fonctions de l'organisme : la croissance (pour le Zn, le F, le Mn, le Cu, le Se, le V), la reproduction (Zn, Mn), la dentition (F, Mo, V), la gustation (Cu, Zn, Ni), l'érythropoïèse (Fe, Zn, Cu, Co, Mo), la coagulation (Mn, Cu, Zn), le métabolisme glucidique (Cr, Mn, Zn), le métabolisme lipidique (Cr, V, Zn, Cu), les échanges cellulaires (Rb, K), etc...

Aussi de nombreuses préparations contenant des oligoéléments et/ou des éléments minéraux ont-elles été préconisées et mises sur le marché dans le cadre d'utilisation thérapeutique, diététique ou dermato-cosmétologique.

Lesdites préparations, proposées dans l'Art antérieur contiennent soit un de ces éléments (préparations monoélémentaires), soit une association de ces éléments (préparations multiélémentaires).

Toutefois, lesdits éléments minéraux et/ou oligoéléments essentiels, désignés ci-après par le terme "éléments", sont le plus souvent utilisés de façon monoélémentaire, et même dans le cas d'associations multiélémentaires, dans un but supplétif d'une ou plusieurs carences. Lorsque le but n'est pas directement supplétif, lesdits éléments sont associés dans leurs indications à des notions d'équilibre de terrain, et non de pathologie ; en particulier, en ce qui concerne les "oligosols", ils contiennent lesdits éléments à des doses faibles, lesdits éléments étant alors associés de façon quelconque ; par ailleurs, pour une application limitée d'une association de K, Rb et Cs au traitement de cellules cancéreuses par PH élevé (BREWER et Al. in MICROBIOS. LETT. 25 (99-100) 1984), lesdits éléments sont également à doses très faibles et associés de façon quelconque.

Un certain nombre d'autres documents décrivent également des compositions comprenant plusieurs éléments et notamment du rubidium (Dictionnaire Vidal 1986, p. 1041, 788 ; Rote liste 1965, p. 772, 1067).

Dans l'Article d'OKADA et Al. (MED. J. THIROSHIMA UNIV., 1987, 35, 1, 36-42), les effets du rubidium sur les récepteurs de la sérotonine sont édudiés en comparaison avec le lithium.

Toutefois, l'utilisation du ribidium en association avec d'autres éléments pour ses propriétés particulières sur les échanges cellulaires n'a jamais été envisagé dans l'Art antérieur.

La présente invention s'est en conséquence donné pour but de pourvoir à un procédé de préparation de compositions comprenant des "éléments" présentant en association des propriétés intéressantes dans les domaines thérapeutique, dermato-cosmétologique et diététique.

C'est, aussi, un but de l'invention de pourvoir à des compositions comprenant des "éléments", notamment du Rb, présentant des propriétés remarquables de traitement curatif ou préventif en médecine humaine ou animale.

Les travaux des Demandeurs ont montrés de façon surprenante qu'il était possible d'obtenir des compositions d'"éléments" qui présentent des propriétés tout à fait inattendues et nouvelles dans leur application à l'organisme vivant, humain ou animal, notamment thérapeutique, dermato-cosmétologique et diététique.

La présente invention a pour objet des compositions d'éléments minéraux, désignées ci-après comme compositions multiélémentaires, caractérisées en ce qu'elles comprennent au moins les éléments $E_1$, $E_2$ ... $E_n$

2

suivants : Rb, K, Mg, Se et Zn -qui constituent la composition multiélémentaire de base-, à des concentrations $C_1$, $C_2$,... $C_n$ telles que les valeurs des rapports de concentration desdites compositions multiélémentaires soient respectivement comprises entre les valeurs limites des rapports :

$$R_1 = \frac{Cx_1}{Cx_1}, R_2 = \frac{Cx_2}{Cx_1}, ... R_n = \frac{Cx_n}{Cx_1}$$

dans lesquels $Cx_1$, ... $Cx_n$ représentent les valeurs des concentrations physiologiques de ces éléments dans un milieu biologique spécifique.

Selon un mode de réalisation avantageux de l'invention, la composition de base renferme au moins un autre élément minéral supplémentaire.

Selon une disposition avantageuse de ce mode de réalisation, lesdits éléments supplémentaires sont à des concentrations telles que définies ci-dessus.

Selon une autre disposition avantageuse de ce mode de réalisation, lesdits éléments supplémentaires sont à des concentrations indépendantes des rapports $R_1$, ... $R_n$ tels que définis ci-dessus.

Selon un autre mode de réalisation avantageux de l'invention, la composition de base renferme au moins un autre élément minéral supplémentaire et/ou un cofacteur.

Selon une disposition préférée de ce mode de réalisation, lesdits éléments supplémentaires sont à des concentrations telles qu'elles présentent des rapports $R_1$, ... $R_n$ tels que définis ci-dessus.

Selon une autre disposition préférée de ce mode de réalisation, lesdits éléments supplémentaires sont à des concentrations indépendantes des rapports $R_1$, ... $R_n$ tels que définis ci-dessus.

Selon encore une autre disposition avantageuse de ce mode de réalisation, les éléments sont sous forme de sels pharmaceutiquement acceptables et compatibles entre eux.

La présente invention a également pour objet un procédé de préparation de compositions telles que définies ci-dessus, destinées à l'administration à une espèce vivante donnée, caractérisé :

– en ce qu'on détermine, pour un milieu biologique spécifique de ladite espèce vivante donnée et pour au moins les éléments minéraux $E_1$, $E_2$, ... $E_n$ en nombre fini suivants : Rb, K, Mg, Se et Zn, les valeurs des concentrations physiologiques $Cx_1$, $Cx_2$, ... $Cx_n$ de ces éléments dans ledit milieu ;

– en ce qu'on détermine les valeurs des rapports

$$R_1 = \frac{Cx_1}{Cx_1}, R_2 = \frac{Cx_2}{Cx_1}, R_n = \frac{Cx_n}{Cx_1},$$

de concentration desdits éléments $E_1$, $E_2$, ..., $E_n$, dans ledit milieu ainsi que les valeurs limites desdits rapports ;

– en ce qu'on prépare une composition multiélémentaire en mélangeant au moins lesdits éléments $E_1$, $E_2$, ... $E_n$ à des concentrations $C_1$, $C_2$, ... $C_n$ telles que les valeurs des rapports de concentration de ladite composition multiélémentaire soient respectivement comprises entre les valeurs limites des rapports $R_1$, $R_2$, ... $R_n$;

– et en ce que l'on met ladite composition sous forme assimilable par ladite espèce.

La présente invention a en outre pour objet des médicaments contenant une composition selon l'invention, dont les "éléments" sont sous forme de sels pharmaceutiquement acceptables et compatibles entre eux.

Lorsque les compositions selon l'invention présentent, outre les éléments Rb, K, Mg, Se et Zn constituant une "composition multiélémentaire de base", un ou des éléments supplémentaires, il a été constaté que ladite composition de base a un rôle synergique sur l'activité dudit ou desdits éléments supplémentaires favorisant une activité de supplémentation dudit/desdits éléments suplémentaires.

Les compositions selon l'invention présentent également une activité de rééquilibration du ou des éléments supplémentaires ajoutés à la composition multiélémentaire de base.

La composition multiélémentaire de base selon l'invention présente une activité de stimulation du métabolisme des cellules adipeuses.

Les compositions selon l'invention peuvent être administrées seules ou en combinaison avec des supports inertes, non toxiques, acceptables du point de vue pharmaceutique, solides, pâteux ou liquides. On peut considérer dans ces formes les comprimés, dragées, capsules, pilules, suspensions et solutions aqueuses, les crèmes, lotions, poudres.

Les demandeurs pensent, sans vouloir être liés par cette indication, que les effets surprenants rapportés ci-dessus sont dus à l'action sur la membrane cellulaire, notamment en modifiant l'échange cellulaire, de certains desdits éléments essentiels et notamment le rubidium, de préférence associé au K, Mg, Se et Zn, comme il sera explicité ci-après.

L'invention sera mieux comprise à l'aide du complément de description qui va suivre, qui se réfère à des exemples de comparaison conformes à l'invention ainsi qu'à des compte-rendus d'expérimentation relatifs à l'activité physiologique desdites compositions conformes à l'invention et des médicaments, produits de cos-

3

métologie, qui en dérivent.

Il doit être bien entendu, toutefois, que ces exemples et compte-rendus sont donnés uniquement à titre d'illustration de l'objet de l'invention, dont ils ne constituent en aucune manière une limitation.

## I - <u>MILIEU BIOLOGIQUE HUMAIN</u>

On sait que chez l'homme sain, le sang contient les "éléments" essentiels précités à des concentrations physiologiques $C_x$ telles que définies ci-après.

| Elément | $C_x$ mg/litre |
|---------|----------------|
| Ca | 91 |
| Mg | 20 |
| Fe | 1,70 |
| Mn | 0,25 |
| Co | 0,30 |
| Ni | 0,30 |
| Cu | 1,05 |
| Zn | 0,95 |
| Cr | |
| Rb | 0,16 |
| Br | 3,60 |
| I | 0,85 |
| Se | 0,08 |
| Mo | 0,025 |
| V | 0,025 |
| K | 173 |

Pour la mise en oeuvre de l'invention, on détermine d'abord les rapports $R_1$, $R_2$ ... $R_n$ au sens donné ci-dessus à ces rapports et l'on obtient ainsi une matrice de rapports de concentrations en fonction de l'élément de référence, une telle matrice étant comme visible ci-dessous :

| $E_n$ | $E_1$ | $E_2$ | $E_3$ | ..... | $E_n$ |
|---|---|---|---|---|---|
| $E_1$ | $R_1 = \dfrac{C_{x1}}{C_{x1}}$ | $R_1 = \dfrac{C_{x2}}{C_{x1}}$ | $R_1 = \dfrac{C_{x3}}{C_{x1}}$ | ..... | $R_1 = \dfrac{C_{xn}}{C_{x1}}$ |
| $E_2$ | $R_2 = \dfrac{C_{x1}}{C_{x2}}$ | $R_2 = \dfrac{C_{x2}}{C_{x2}}$ | $R_2 = \dfrac{C_{x3}}{C_{x2}}$ | ..... | $R_2 = \dfrac{C_{xn}}{C_{x2}}$ |
| $E_3$ | $R_3 = \dfrac{C_{x1}}{C_{x3}}$ | $R_3 = \dfrac{C_{x2}}{C_{x3}}$ | $R_3 = \dfrac{C_{x3}}{C_{x3}}$ | ..... | $R_3 = \dfrac{C_{xn}}{C_{x3}}$ |
| $\vdots$ | $\vdots$ | $\vdots$ | $\vdots$ | ..... | $\vdots$ |
| $E_n$ | $R_n = \dfrac{C_{x1}}{C_{xn}}$ | $R_n = \dfrac{C_{x2}}{C_{xn}}$ | $R_n = \dfrac{C_{x3}}{C_{xn}}$ | ..... | $R_n = \dfrac{C_{xn}}{C_{xn}}$ |

Dans cette matrice :

$E_n$ désigne l'élément $E_n$ présent dans le sérum.

$C_{xn}$ désigne la concentration de l'élément $E_1$, $E_2$ ... $E_n$ dans le sérum.

R désigne le rapport des concentrations de l'élément $E_1/E_n$ dans le sérum, soit $\dfrac{Cx_1}{Cx_n}$

Chez l'homme, ces rapports ont les valeurs suivantes en référence au calcium.

| Elément | Rapport |
|---------|---------|
| Ca | 1 |
| Mg | 0,79 |
| Fe | 0,22 |
| Mn | $1,1.10^{-3}$ |
| Co | $0,55.10^{-3}$ |
| Ni | $0,55.10^{-3}$ |
| Cu | $1,4.10^{-2}$ |
| Zn | $7,2.10^{-2}$ |
| Cr | $4,5.10^{-4}$ |
| Rb | $2,4.10^{-2}$ |
| Br | $6,1.10^{-2}$ |
| I | $1.10^{-2}$ |
| Se | $0,8.10^{-3}$ |
| Mo | $2,8.10^{-4}$ |
| V | $2,8.10^{-4}$ |
| K | $3,5.10^{-2}$ |

Si, pour une composition multiélémentaire donnée, l'ensemble ou la majorité des rapports homologues entre le milieu biologique, tel que le sang par exemple, et ladite composition, sont identiques, ladite composition est conforme à l'invention.

Les variations extrêmes des concentrations et des rapports sont définies comme suit :

La concentration physiologique d'un élément est comprise entre -5 et +5 Ecart-Types par rapport à la moyenne. Les rapports extrêmes physiologiques pour deux éléments A et B, si Am et Bm sont les concentrations moyennes dans le milieu biologique de référence de A et B, et si ETA et ETB sont les valeurs de la déviation standard à la moyenne de A et B pour ce milieu, les valeurs extrêmes physiologiques du rapport entre A et B seront :

$$\frac{Am + 5\,ETA}{Bm - 5\,ETA} \text{ et } \frac{Am - 5\,ETA}{Bm + 5\,ETA}$$

A partir de ces valeurs, on prépare ensuite la composition standard suivante, lorsque l'on prend le calcium (Ca) comme élément de référence.

| Elément | $C_n$ |
|---------|-------|
| Ca | 0,353 g |
| Mg | 0,28 g |
| Fe | 80 mg |
| Mn | 0,4 mg |
| Co | 0,2 mg |
| Ni | 0,2 mg |
| Cu | 5 mg |
| Zn | 25,4 mg |
| Cr | 160 μg |
| Rb | 8,48 mg |
| Br | 21,6 mg |
| I | 3,56 mg |
| Se | 0,28 mg |
| Mo | 0,1 mg |
| V | 0,1 mg |
| K | 12,3 mg |

N.B : Les concentrations sont exprimées en masse

## II - MILIEU BIOLOGIQUE ANIMAL

**Exemples de variations des rapports interélémentaires interéquilibrés physiologiques chez différentes espèces, en considérant le calcium comme référence**

| Elément | ESPECE | | |
|---|---|---|---|
| | CHIEN | CHAT | CHEVAL |
| Se | $6.10^{-3}$ | $3,2.10^{-3}$ | $0,3.10^{-3}$ |
| Rb | $2,4.10^{-2}$ | $4.10^{-2}$ | $2.10^{-2}$ |
| Zn | $6.10^{-2}$ | $6.10^{-2}$ | $3,6.10^{-2}$ |
| Mg | . | . | . |
| K | . | . | . |

Pour réaliser une composition multiélémentaire équilibrée conforme à l'invention et physiologique, pour une espèce donnée, les rapports ci-dessus devront être respectés.

## IV - APPLICATIONS THERAPEUTIQUES

### IV.1. TRAITEMENTS DE SUPPLEMENTATION

L'élément à supplémenter est à des doses non comprises dans les rapports tels que définis, les autres éléments étant présents à des concentrations telles que les rapports interélémentaires conformes à l'invention soient respectés.

**Exemple 1 - Potassium**

|  | Potassium injectable (KCl à 10 %) | Potassium comprimés (KCl à 1 g/comprimé) |
|---|---|---|
| Mg | 2 mg | 20,6 mg |
| Zn | 0,1 mg | 0,94 mg |
| Rb | 0,21 mg | 2,1 mg |
| Se | 0,01 mg | 0,08 mg |
| K | 5,24 g | 0,524 g |

Mg, Zn, Rb et K sont sous forme de chlorure.

Se est sous forme de sélénite de sodium.

**Exemple 2 - Calcium**

|  | Calcium injectable (pour 1 ampoule de 10mg) | Calcium comprimé |
|---|---|---|
| Ca | 180 mg | 150 mg |
| Mg | 0,2 mg | 20,6 mg |
| Zn | 0,01 mg | 0,94 mg |
| Rb | 0,02 mg | 2,1 mg |
| Se | 0,001 mg | 0,08 mg |
| K | 1,95 mg | 195 mg |

Le calcium est sous forme de gluconate.

Mg, Zn, Rb, K sont sous forme de chlorure.

Se est sous forme de sélénite de sodium.

**Exemple 3 - Magnésium**

|  | Magnésium injectable (pour 1 ampoule de 10ml) | Magnésium comprimés (pour 1 g/comprimé) |
|---|---|---|
| Mg | 100 mg | 50 mg |
| Zn | 0,01 mg | 0,94 mg |
| Rb | 0,02 mg | 2,1 mg |
| Se | 0,001 mg | 0,08 mg |
| K | 1,95 mg | 195 mg |

Zn, Rb, K sont sous forme de chlorure.

Mg est sous forme de chlorure de lactate.

Se est sous forme de sélénite de sodium.

**Exemple 4 - Fer**

|  | Fer comprimé (pour 1 g/comprimé) |
|---|---|
| Mg | 20,6 mg |
| Fe | 100 mg |
| Zn | 0,94 mg |
| Rb | 2,1 mg |
| Se | 0,08 mg |
| K | 195 mg |
| Vit C | 500 mg |

Fe : Chlorure ou sulfate.

**Exemple 5 - Fluor**

| | Fluor comprimé (pour 1 g/comprimé) |
|---|---|
| Mg | 20,6 mg |
| Zn | 0,94 mg |
| Rb | 2,1 mg |
| Se | 0,08 mg |
| K | 195 mg |
| F | 0,5 mg |

**Exemple 6 - Sélénium**

| | Sélénium comprimé (pour 1 g/comprimé) |
|---|---|
| Mg | 20,6 mg |
| Zn | 0,94 mg |
| Rb | 2,1 mg |
| Se | 0,3 mg |
| K | 195 mg |

**Exemple 7 - Chrome**

| | Chrome comprimé (pour 1 g/comprimé) |
|---|---|
| Mg | 20,6 mg |
| Zn | 0,95 mg |
| Cr | 0,005 mg |
| Rb | 2,1 mg |
| Se | 0,08 mg |
| K | 195 mg |

## IV.2. TRAITEMENT DE REEQUILIBRATION

Les rapports interélémentaires doivent être modifiés en fonction de la pathologie dont il faut interéquilibrer les éléments.

A titre d'exemple, pour les pathologies suivantes, il faudra utiliser les rapports :

| ELEMENT | ARTHROSE | HYPERTENSION | DERMATOLOGIE |
|---------|----------|--------------|--------------|
| Mg | 0,6 | 0,6 | 0,6 |
| Zn | $6.10^{-2}$ | $6.10^{-2}$ | $5.10^{-2}$ |
| Se | $0,7.10^{-3}$ | Normal | Normal |
| Rb | Normal | $1,8.10^{-2}$ | Normal |
| K | Normal | Normal | Normal |

## Exemple 8

| PATHOLOGIE | Ca | Mg | Fe | Mn | Co | Ni | Cu | Zn | Cr | Rb | Br | I | Se | Mo | V | K |
|---|---|---|---|---|---|---|---|---|---|---|---|---|---|---|---|---|
| STANDARD | 6 | 1,40 | 1,3 | 0,03 | 0,03 | 0,03 | 0,083 | 0,062 | 0,003 | 0,14 | 0,36 | 0,06 | 0,005 | 0,001 | 0,0007 | 13 |
| OS-ARTICULATION | 8,4 | 1,10 | 1,82 | 0,03 | 0,03 | 0,03 | 0,083 | 0,087 | 0,003 | 0,112 | 0,36 | 0,084 | 0,003 | 0,001 | 0,0007 | 13 |
| GASTRO-INTESTINALE | 10,8 | 1,68 | 1,30 | 0,03 | 0,03 | 0,03 | 0,099 | 0,087 | 0,003 | 0,140 | 0,57 | 0,11 | 0,005 | 0,001 | 0,0007 | 13 |
| ENDOCRINOLOGIE | 8,4 | 1,40 | 1,56 | 0,03 | 0,03 | 0,03 | 0,066 | 0,099 | 0,003 | 0,140 | 0,36 | 0,096 | 0,003 | 0,001 | 0,0007 | 13 |
| CARDIO-VASCULAIRE | 8,4 | 0,84 | 1,56 | 0,03 | 0,03 | 0,03 | 0,083 | 0,086 | 0,003 | 0,140 | 0,36 | 0,06 | 0,005 | 0,001 | 0,0007 | 13 |
| DERMATOLOGIE | 2,4 | 0,84 | 1,56 | 0,03 | 0,03 | 0,03 | 0,100 | 0,086 | 0,003 | 0,140 | 0,57 | 0,084 | 0,005 | 0,001 | 0,0007 | 13 |
| NEUROLOGIE | 2,4 | 1,70 | 1,30 | 0,03 | 0,03 | 0,03 | 0,083 | 0,074 | 0,003 | 0,197 | 0,43 | 0,036 | 0,003 | 0,001 | 0,0007 | 13 |
| INFECTIONS | 8,4 | 0,84 | 1,82 | 0,03 | 0,03 | 0,03 | 0,133 | 0,111 | 0,003 | 0,112 | 0,216 | 0,095 | 0,007 | 0,001 | 0,0007 | 13 |
| ASTHENIE-DEPRESSION | 7,2 | 1,40 | 1,80 | 0,03 | 0,03 | 0,03 | 0,116 | 0,087 | 0,003 | 0,200 | 0,36 | 0,072 | 0,004 | 0,001 | 0,0007 | 13 |
| MIGRAINES | 9,6 | 1,40 | 1,56 | 0,03 | 0,03 | 0,03 | 0,083 | 0,099 | 0,003 | 0,22 | 0,36 | 0,072 | 0,004 | 0,001 | 0,0007 | 13 |
| CIRCULATION | 6 | 0,56 | 1,30 | 0,03 | 0,03 | 0,03 | 0,099 | 0,074 | 0,003 | 0,14 | 0,28 | 0,06 | 0,004 | 0,001 | 0,0007 | 13 |
| HYPERTENSION | 7,2 | 0,56 | 1,82 | 0,03 | 0,03 | 0,03 | 0,050 | 0,087 | 0,003 | 0,168 | 0,36 | 0,072 | 0,004 | 0,001 | 0,0007 | 13 |
| DIABETE | 8,4 | 1,12 | 1,04 | 0,03 | 0,03 | 0,03 | 0,083 | 0,111 | 0,003 | 0,14 | 0,50 | 0,09 | 0,002 | 0,001 | 0,0007 | 13 |
| OBESITE | 8,4 | 1,68 | 1,82 | 0,03 | 0,03 | 0,03 | 0,066 | 0,087 | 0,003 | 0,140 | 0,290 | 0,084 | 0,004 | 0,001 | 0,0007 | 13 |
| ARTHROSE | 6 | 0,56 | 1,30 | 0,03 | 0,03 | 0,03 | 0,083 | 0,074 | 0,003 | 0,14 | 0,290 | 0,096 | 0,002 | 0,001 | 0,0007 | 13 |
| ALLERGIE | 6 | 1,40 | 1,56 | 0,03 | 0,03 | 0,03 | 0,133 | 0,087 | 0,003 | 0,224 | 0,360 | 0,072 | 0,007 | 0,001 | 0,0007 | 13 |

en mg et calculé pour une dose administrable par voie orale.

## V - APPLICATION A LA MESOTHERAPIE ET A LA DERMATO-COSMETOLOGIE

### Exemple 9 - Solution de stimulation du métabolisme des cellules adipeuses

```
Mg   0,1 à 1 mg
Zn   0,1 - 1 mg
Rb   0,04 - 0,4 mg
K    0,1 - 0,5 mg
Cr   0,007 - 0,01 mg

                    qsp amp. 2 ml
```

### Exemple 10 - Pommade utilisée comme draineur du tissu adipeux

```
Mg   5 à 50 mg
Zn   5 - 50 mg
Rb   2 - 40 mg
K    2 - 40 mg
Se   1 - 10 mg

                    qsp 1 g de pommade
```

## VI - TESTS PHYSIOLOGIQUES

**Exemple A**

La composition de base (Rb, K, Se, Zn, Mg) a été élaborée et testée en étudiant la résistance globulaire sur des globules rouges humains, et/ou de chiens, et/ou de chats (Test de résistance globulaire).

### 1. Principes de la technique utilisée

Les globules rouges (GR) sont mis en présence de solutions de chlorure de sodium à concentration décroissante (solution de départ isotonique puis solutions hypotoniques par dilutions successives). Cette méthode permet de mesurer, pour une population donnée de globules rouges (GR), la résistance membranaire de la cellule.

### 2. Détermination qualitative

Les éléments suivants : rubidium, potassium, sélénium, zinc, magnésium, ont tout d'abord été testés, l'un après l'autre, en utilisant une solution de départ isotonique comprenant l'un des éléments seul en milieu NaCl à des concentrations voisines de celles rencontrées dans le plasma de l'espèce concernée.

Ces épreuves étaient faites simultanément avec une série témoin ne comprenant que du chlorure de sodium. Il a été constaté une résistance augmentée avec chacun des éléments précédents.

| | |
|---|---|
| Rb | + + |
| K | + |
| Se | + |
| Zn | + |
| Mg | + |

$$(+) = \text{au moins un tube de décalage}$$
$$(++) = \text{2 ou 3 tubes de décalage}$$
$$(+++) = \text{supérieur à 3 tubes de décalage}$$

Conclusion

Les éléments précités possèdent une activité individuelle sur la résistance membranaire et les activités de membrane.

Il a ensuite été procédé à la même expérience en associant les éléments deux par deux, puis trois par trois, jusqu'à l'utilisation des cinq éléments simultanés.

Les résultats obtenus dans les associations partielles ne dépassaient pas celles du rubidium seul, et seul le mélange des cinq éléments permettait d'obtenir un résultat supérieur (+++).

**3. Détermination quantitative**

Les concentrations de chacun des éléments ont alors été modifiées, séparément pour voir si la réponse était améliorée.

Une amélioration légère a été obtenue en augmentant la concentration en rubidium.

Par contre les modifications significatives des autres éléments avaient un effet négatif. Les proportions interélémentaires du mélange sont les suivantes :

| ELEMENT | HOMME | CHIEN | CHAT | CHEVAL |
|---|---|---|---|---|
| Rb | 2,1 | 2,1 | 4 | 1,7 |
| K | 195 | 195 | 195 | 195 |
| Se | 0,08 | 0,06 | 0,032 | 0,03 |
| Zn | 0,94 | 0,60 | 0,60 | 0,40 |
| Mg | 20,6 | 32 | 32 | 27 |

(mg/l)

**4. Modifications de concentration**

Le même protocole a été réutilisé en augmentant, dans le liquide physiologique, la proportion de la composition de base, comme indiqué dans le Tableau ci-après.

### LIMITES DE CONCENTRATION POUR L'HOMME (mg/l)

| Elément | Dilution | | | | | | Base | Concentration | | | | |
|---|---|---|---|---|---|---|---|---|---|---|---|---|
| | 1/64 | 1/32 | 1/16 | 1/8 | 1/4 | 1/2 | 1/1 | x2 | x3 | x4 | x5 | x6 |
| Rb | 0,03 | 0,06 | 0,14 | 0,27 | 0,53 | 1,05 | 2,1 | 4,2 | 6,3 | 8,39 | 10,5 | 12,6 |
| K | 3,13 | 6,26 | 12,5 | 25,7 | 49,2 | 97,5 | 195 | 390 | 585 | 780 | 975 | 1170 |
| Se | 0,001 | 0,002 | 0,005 | 0,01 | 0,02 | 0,04 | 0,08 | 0,16 | 0,24 | 0,32 | 0,40 | 0,48 |
| Zn | 0,01 | 0,03 | 0,06 | 0,12 | 0,23 | 0,47 | 0,94 | 1,88 | 2,82 | 3,76 | 4,70 | 5,64 |
| Mg | 0,33 | 0,66 | 1,32 | 2,64 | 5,19 | 10,3 | 20,6 | 41,2 | 61,8 | 82,4 | 103 | 123 |

Chlorure de sodium et $H_2O$ QSP un litre isotonique

Résultats :

Les résultats restaient semblables entre une dilution au quart et une concentration X 3.

### 5. Conclusion

La composition citée a un rôle sur la protection de la membrane qui se traduit par une résistance augmentée des cellules à l'hémolyse.

### Exemple B

### 1. Protocole

L'activation des échanges cellulaires pour la composition multiélémentaire de base a été testée. Des cellules vivantes (spermatozoïdes humains) ont été utilisées. Les spermatozoïdes vivants ont la propriété de capter les colorants vitaux. On a constaté en présence de ladite composition, que le colorant pénétrait plus rapidement à l'intérieur des cellules.

De plus, sur des spermatozoïdes provenant du même donneur, il a été procédé à la même expérience en laissant les spermatozoïdes 4 heures et 8 heures, et il a été constaté que le nombre de spermatozoïdes survivants en présence de ladite composition était supérieur à celui de l'expérience témoin.

### 2. Conclusion

La composition multiélémentaire de base qui comprend Rb, K, Se, Mg, Zn, favorise l'échange cellulaire.

### Exemple C

### Utilisation in-vivo

La cellule adipeuse (adipocyte) est une cellule à métabolisme ralenti, la cellule s'engorge, se draine mal et c'est ainsi que s'installe la cellulite dont le dernier stade est l'intoxication complète de la cellule adipeuse qui aboutit à une fibrose. Un soluté injectable dont la composition est :

| Rb | 2,1 |
|----|-----|
| K | 195 |
| Se | 0,08 |
| Zn | 0,94 |
| Mg | 20,6 |

(mg/l)

1 dose = 2 ml

a été testé.

Cette solution a été injectée localement selon la méthode classique de la mésothérapie, chez 35 patientes selon le protocole suivant : multi-injections locales à raison d'une séance par semaine pendant 6 semaines. Pour chaque patiente, des mesures du panicule adipeux ont été effectuées selon les principes de mesure traditionnels et avec de plus un contrôle échographique.

**Résultats**

| Amélioration de l'aspect de la peau (peau d'orange) | 34 | | | |
|-----|-----|-----|-----|-----|
| Diminution mesurable du panicule adipeux | Importante | Moyenne | Faible | Abandon |
| | 7 | 20 | 7 | 1 |

**Conclusion**

La composition injectée a bien provoqué, localement, une activation des échanges cellulaires des adipocytes qui s'est traduite par un drainage de ces cellules et une diminution du panicule adipeux.

La composition a donc bien eu des propriétés activatrices au niveau des échanges membranaires cellulaires.

**Revendications**

**Revendications pour les Etats contractants suivants : AT, BE, CH, DE, FR, GB, IT, LI, LU, NL, SE.**

1. Compositions d'éléments minéraux, désignées ci-après comme compositions multiélémentaires, caractérisées en ce qu'elles comprennent au moins les éléments $E_1$, $E_2$ ... $E_n$ suivants : Rb, K, Mg, Se et Zn -qui constituent la composition multiélémentaire de base-, à des concentrations $C_1$, $C_2$,... $C_n$ telles que les valeurs des rapports de concentration desdites compositions multiélémentaires soient respectivement comprises entre les valeurs limites des rapports :

$$R_1 = \frac{Cx_1}{Cx_1}, R_2 = \frac{Cx_2}{Cx_1}, ... R_n = \frac{Cx_n}{Cx_1}$$

dans lesquels $Cx_1$, ... $Cx_n$ représentent les valeurs des concentrations physiologiques de ces éléments dans

# EP 0 306 377 B1

un milieu biologique spécifique.

2. Compositions selon la revendication 1, caractérisées en ce que la composition de base renferme au moins un autre élément minéral supplémentaire.

3. Compositions selon la revendication 2, caractérisées en ce que lesdits éléments supplémentaires sont à des concentrations selon la revendication 1.

4. Compositions selon la revendication 2, caractérisées en ce que lesdits éléments supplémentaires sont à des concentrations indépendantes des rapports $R_1, ... R_n$ selon la revendication 1.

5. Compositions selon la revendication 1, caractérisées en ce que la composition de base renferme au moins un autre élément minéral supplémentaire et/ou un cofacteur.

6. Compositions selon la revendication 5, caractérisées en ce qui lesdits éléments supplémentaires sont à des concentrations telles qu'elles présentent des rapports $R_1, ... R_n$ selon la revendication 1.

7. Compositions selon la revendication 5, caractérisées en ce que lesdits éléments supplémentaires sont à des concentrations indépendantes des rapports $R_1, ... R_n$ selon la revendication 1.

8. Compositions selon l'une quelconque des revendications 1 à 7, caractérisées en ce que les éléments sont sous forme de sels pharmaceutiquement acceptables et compatibles entre eux.

9. Procédé de préparation de compositions selon l'une des revendications 1 à 9, destinées à l'administration à une espèce vivante donnée, caractérisé :

– en ce qu'on détermine, pour un milieu biologique spécifique de ladite espèce vivante donnée et pour au moins les éléments minéraux $E_1, E_2, ... E_n$ en nombre fini suivants : Rb, K, Mg, Se et Zn, les valeurs des concentrations physiologiques $Cx_1, Cx_2, ... Cx_n$ de ces éléments dans ledit milieu ;

– en ce qu'on détermine les valeurs des rapports

$$R_1 = \frac{Cx_1}{Cx_1}, R_2 = \frac{Cx_2}{Cx_1}, R_n = \frac{Cx_n}{Cx_1},$$

de concentration desdits éléments $E_1, E_2, ..., E_n$, dans ledit milieu ainsi que les valeurs limites desdits rapports ;

– en ce qu'on prépare une composition multiélémentaire en mélangeant au moins lesdits éléments $E_1, E_2, ... E_n$ à des concentrations $C_1, C_2, ... C_n$ telles que les valeurs des rapports de concentration de ladite composition multiélémentaire soient respectivement comprises entre les valeurs limites des rapports $R_1, R_2, ... R_n$;

– et en ce que l'on met ladite composition sous forme assimilable par ladite espèce.

10. Utilisation des compositions selon l'une quelconque des revendications 1 à 8, pour l'obtention d'un médicament destiné à favoriser l'activité de supplémentation du/des éléments supplémentaire(s) ajouté(s) à la composition multiélémentaire de base.

11. Utilisation des compositions selon l'une quelconque des revendications 1 à 8, pour l'obtention d'un médicament destiné à activer la rééquilibration du/des élément(s) supplémentaire(s) ajouté(s) à la composition multiélémentaire de base.

12. Utilisation des compositions selon l'une quelconque des revendications 1 à 8, pour l'obtention d'un médicament destiné à stimuler le métabolisme des cellules adipeuses.

**Revendications pour les Etats contractants suivants : ES, GR**

1. Procédé de préparation de compositions d'éléments minéraux, désignées ci-après compositions multiélémentaires, destinées à l'administration à une espèce vivante donnée, caractérisé :

– en ce qu'on détermine, pour un milieu biologique spécifique de ladite espèce vivante donnée et pour au moins les éléments minéraux $E_1, E_2, ... E_n$ en nombre fini suivants : Rb, K, Mg, Se et Zn, les valeurs des concentrations physiologiques $Cx_1, Cx_2, ... Cx_n$ de ces éléments dans ledit milieu ;

– en ce qu'on détermine les valeurs des rapports

$$R_1 = \frac{Cx_1}{Cx_1}, R_2 = \frac{Cx_2}{Cx_1}, R_n = \frac{Cx_n}{Cx_1},$$

de concentration desdits éléments $E_1, E_2, ..., E_n$, dans ledit milieu ainsi que les valeurs limites desdits rapports ;

– en ce qu'on prépare une composition multiélémentaire en mélangeant au moins lesdits éléments $E_1, E_2, ... E_n$ à des concentrations $C_1, C_2, ... C_n$ telles que les valeurs des rapports de concentration de ladite composition multiélémentaire soient respectivement comprises entre les valeurs limites des rapports $R_1, R_2, ... R_n$;

– et en ce que l'on met ladite composition sous forme assimilable par ladite espèce.

2. Procédé selon la revendication 1, caractérisé en ce la composition de base renferme au moins un autre élément minéral supplémentaire.

3. Procédé selon la revendication 2, caractérisé en ce que lesdits éléments supplémentaires sont à des concentrations selon la revendication 1.

4. Procédé selon la revendication 2, caractérisé en ce que lesdits éléments supplémentaires sont à des concentrations indépendantes du rapports $R_1, ... R_n$ selon la revendication 1.

5. Procédé selon la revendication 1, caractérisé en ce que la composition de base renferme au moins un autre élément minéral supplémentaire et/ou un cofacteur

6. Procédé la revendication 5, caractérisé en ce qui lesdits éléments supplémentaires sont à des concentrations telles qu'elles présentent des rapports $R_1, ... R_n$ selon la revendication 1.

7. Procédé selon la revendication 5, caractérisé en ce que lesdits éléments supplémentaires sont à des concentrations indépendantes des rapports $R_1, ... R_n$ selon la revendication 1.

8. Procédé selon l'une quelconque des revendications 1 à 7, caractérisé en ce que les éléments sont sous forme de sels pharmaceutiquement acceptables et compatibles entre eux.

9. Utilisation des compositions selon l'une quelconque des revendications 1 à 8, pour l'obtention d'un médicament destiné à favoriser l'activité de supplémentation du/des éléments supplémentaire(s) ajouté(s) à la composition multiélémentaire de base.

10. Utilisation des compositions selon l'une quelconque des revendications 1 à 8, pour l'obtention d'un médicament destiné à activer la rééquilibration du/des élément(s) supplémentaire(s) ajouté(s) à la composition multiélémentaire de base.

11. Utilisation des compositions selon l'une quelconque des revendications 1 à 8, pour l'obtention d'un médicament destiné à stimuler le métabolisme des cellules adipeuses.

## Patentansprüche

**Patentansprüche für folgende Vertragsstaaten : AT, BE, CH, DE, FR, GB, IT, LI, LU, NL, SE**

1. Zusammensetzungen aus mineralischen Elementen, nachstehend als Multielement-Zusammensetzungen bezeichnet, dadurch gekennzeichnet, daß sie enthalten mindestens die folgenden Elemente $E_1, E_2...E_n$: Rb, K, Mg, Se und Zn - welche die Multielement-Grundzusammensetzung darstellen - in solchen Konzentrationen $C_1, C_2, ... C_n$, daß die Werte der Konzentrationsverhältnisse dieser Multielement-Zusammensetzungen jeweils zwischen den Grenzwerten der Verhältnisse liegen:

$$R_1 = \frac{Cx_1}{Cx_1}, R_2 = \frac{Cx_2}{Cx_1}, ... R_n = \frac{Cx_n}{Cx_1}$$

worin $Cx_1, ... Cx_n$ die Werte der physiologischen Konzentrationen dieser Elemente in einem spezifischen biologischen Milieu darstellen.

2. Zusammensetzungen nach Anspruch 1, dadurch gekennzeichnet, daß die Grundzusammensetzung mindestens ein anderes ergänzendes (zusätzliches) mineralisches Element enthält.

3. Zusammensetzungen nach Anspruch 2, dadurch gekennzeichnet, daß die ergänzenden (zusätzlichen) Elemente in Konzentrationen gemäß Anspruch 1 vorliegen.

4. Zusammensetzungen nach Anspruch 2, dadurch gekennzeichnet, daß die ergänzenden (zusätzlichen) Elemente in Konzentrationen vorliegen, die unabhängig von den Verhältnissen $R_1 ... R_n$ gemäß Anspruch 1 sind.

5. Zusammensetzungen nach Anspruch 1, dadurch gekennzeichnet, daß die Grundzusammensetzung mindestens ein anderes ergänzendes (zusätzliches) mineralisches Element und/oder einen Cofaktor enthält.

6. Zusammensetzungen nach Anspruch 5, dadurch gekennzeichnet, daß die ergänzenden (zusätzlichen) Elemente in solchen Konzentrationen vorliegen, daß sie in den Verhältnissen $R_1, ... R_n$ gemäß Anspruch 1 vorliegen.

7. Zusammensetzungen nach Anspruch 5, dadurch gekennzeichnet, daß die ergänzenden (zusätzlichen) Elemente in Konzentrationen vorliegen, die unabhängig von den Verhältnissen $R_1, ... R_n$ gemäß Anspruch 1 sind.

8. Zusammensetzungen nach einem der Ansprüche 1 bis 7, dadurch gekennzeichnet, daß die Elemente in Form von pharmazeutisch akzeptablen und untereinander kompatiblen Salzen vorliegen.

9. Verfahren zur Herstellung der Zusammensetzungen nach einem der Ansprüche 1 bis 8, die bestimmt sind für die Verabreichung an ein gegebenes Lebewesen (Spezies), dadurch gekennzeichnet,

– daß man für ein spezifisches biologisches Milieu des gegebenen Lebewesens (Spezies) und für mindestens die folgenden mineralischen Elemente $E_1, E_2, ... E_n$ in einer endlischen Anzahl: Rb, K, Mg, Se und Zn, die Werte der physiologischen Konzentrationen $Cx_1$, $Cx_2$, ... $Cx_n$ dieser Elemente in diesem Milieu bestimmt;

– daß man die Werte der Konzentrationsverhältnisse dieser Elemente $E_1$, $E_2$, ... $E_n$

$$R_1 = \frac{Cx_1}{Cx_1}, R_2 = \frac{Cx_2}{Cx_1}, R_n = \frac{Cx_n}{Cx_1}$$

in diesem Milieu sowie die Grenzwerte dieser Verhältnisse bestimmt;

– daß man eine Multielement-Zusammensetzung herstellt, indem man mindestens die Elemente $E_1$, $E_2$, ... $E_n$ miteinander mischt in solchen Konzentrationen $C_1$, $C_2$, ... $C_n$, daß die Werte der Konzentrationsverhältnisse der Multielement-Zusammensetzung jeweils zwischen den Grenzwerten der Verhältnisse $R_1$, $R_2$, ... $R_n$ liegen;

– und daß man diese Zusammensetzung in eine für das genannte Lebewesen (Spezies) assimilierbare Form überführt.

10. Verwendung der Zusammensetzungen nach einem der Ansprüche 1 bis 9 zur Herstellung eines Arzneimittels, das dazu bestimmt ist, die Ergänzungsaktivität des/der ergänzenden Elements (Elemente), das (die) der Multielement-Grundzusammensetzung zugefügt worden ist (sind), zu fördern.

11. Verwendung der Zusammensetzungen nach einem der Ansprüche 1 bis 8 zur Herstellung eines Arzneimittels, das dazu besimmt ist, die Reäquilibrierung des/der ergänzenden Elements (Elemente), das (die) der Multielement-Grundzusammensetzung zugefügt worden ist (sind), zu aktivieren.

12. Verwendung der Zusammensetzungen nach einem der Ansprüche 1 bis 8 zur Herstellung eines Arzneimittels, das dazu bestimmt ist, den Stoffwechsel der Fettzellen zu stimulieren.

**Patentansprüche für folgende Vertragsstaaten : ES, GR**

1. Verfahren zur Herstellung von Zusammensetzungen aus mineralischen Elementen, nachstehend als Multielement-Zusammensetzungen bezeichnet, die bestimmt sind für die Verabreichung an ein gegebenes Lebewesen (Spezies), dadurch gekennzeichnet,

– daß man für ein spezifisches biologisches Milieu des gegebenen Lebewesens (Spezies) und für mindestens die folgenden mineralischen Elemente $E_1$, $E_2$, ... $E_n$ in einer endlichen Anzahl: Rb, K, Mg, Se und Zn, die Werte der physiologischen Konzentrationen $Cx_1$, $Cx_2$, ... $Cx_n$ dieser Elemente in diesem Milieu bestimmt;

– daß man die Werte der Konzentrationsverhältnisse dieser Elemente $E_1$, $E_2$, ... $E_n$

$$R_1 = \frac{Cx_1}{Cx_1}, R_2 = \frac{Cx_2}{Cx_1}, R_n = \frac{Cx_n}{Cx_1}$$

in diesem Milieu sowie die Grenzwerte dieser Verhältnisse bestimmt;

– daß man eine Multielement-Zusammensetzung herstellt, indem man mindestens die Elemente $E_1$, $E_2$, ... $E_n$ miteinander mischt in solchen Konzentrationen $C_1$, $C_2$, ... $C_n$, daß die Werte der Konzentrationsverhältnisse der Multielement-Zusammensetzung jeweils zwischen den Grenzwerten der Verhältnisse $R_1$, $R_2$, ... $R_n$ liegen; und

– daß man diese Zusammensetzung in eine für dieses Lebewesen (Spezies) assimilierbare Form überführt.

2. Verfahren nach Anspruch 1, dadurch gekennzeichnet, daß die Grundzusammensetzung mindestens ein anderes ergänzendes (zusätzliches) mineralisches Element enthält.

3. Verfahren nach Anspruch 2, dadurch gekennzeichnet, daß die ergänzenden (zusätzlichen) Elemente in den Konzentrationen gemäß Anspruch 1 vorliegen.

4. Verfahren nach Anspruch 2, dadurch gekennzeichnet, daß die ergänzenden (zusätzlichen) Elemente in Konzentrationen vorliegen, die unabhängig von den Verhältnissen $R_1$, ... $R_n$ gemäß Anspruch 1 sind.

5. Verfahren nach Anspruch 1, dadurch gekennzeichnet, daß die Grundzusammensetzung mindestens ein anderes ergänzendes (zusätzliches) mineralisches Element und/oder einen Cofaktor enthält.

6. Verfahren nach Anspruch 5, dadurch gekennzeichnet, daß die ergänzenden (zusätzlichen) Elemente in solchen Konzentrationen vorliegen, daß sie die Verhältnisse $R_1$, ... $R_n$ gemäß Anspruch 1 darstellen.

7. Verfahren nach Anspruch 5, dadurch gekennzeichnet, daß die ergänzenden (zusätzlichen) Elemente in Konzentrationen vorliegen, die unabhängig von den Verhältnissen $R_1$, ... $R_n$ gemäß Anspruch 1 sind.

8. Verfahren nach einem der Ansprüche 1 bis 7, dadurch gekennzeichnet, daß die Elemente in Form von pharmazeutisch akzeptablen und untereinander kompatiblen Salzen vorliegen.

9. Verwendung der Zusammensetzungen nach einem der Ansprüche 1 bis 8 zur Herstellung eines Arzneimittels, das dazu bestimmt ist, die Ergänzungsaktivität des/der ergänzenden Elements (Elemente), das (die) der Multielement-Grundzusammensetzung zugefügt worden ist (sind), zu fördern.

10. Verwendung der Zusammensetzungen nach einem der Ansprüche 1 bis 8 zur Herstellung eines Arzneimittels, das dazu bestimmt ist, die Reäquilibrierung des/der ergänzenden Elements (Elemente), das (die) der Multielement-Grundzusammensetzung zugefügt worden ist (sind), zu aktivieren.

11. Verwendung der Zusammensetzungen nach einem der Ansprüche 1 bis 8 zur Herstellung eines Arzneimittels, das dazu bestimmt ist, den Stoffwechsel der Fettzellen zu stimulieren.


## Claims

## Claims for the following Contracting States : AT, BE, CH, DE, FR, GB, IT, LI, LU, NL, SE

1. Composition of mineral elements, designated below as multi-element compositions, characterized in that they comprise at least the elements $E_1$, $E_2$ ...... $E_n$ as follows: Rb, K, Mg, Se and Zn, which constitute the basic multi-element composition, in concentrations $C_1$, $C_2$ ...... $C_n$ such that the values of the concentration ratios of the said multi-element compositions lie respectively within the limit values of the ratios:

$$R_1 = \frac{Cx_1}{Cx_1} \quad R_2 = \frac{Cx_2}{Cx_1} \quad ...... \quad R_n = \frac{Cx_n}{Cx_1}$$

in which $Cx_1$, $Cx_2$ ...... $Cx_n$ represent the values of the physiological concentrations of these elements in a specific biological medium.

2. Compositions according to Claim 1, characterized in that the basic composition contains at least one other supplementary mineral element.

3. Compositions according to Claim 2, characterized in that the said supplementary elements are in concentrations according to Claim 1.

4. Compositions according to Claim 2, characterized in that the said supplementary elements are in concentrations independent of the ratios $R_1$, $R_2$ ...... $R_n$ according to Claim 1.

5. Compositions according to Claim 1, characterized in that the basic composition contains at least one other supplementary mineral element and/or a co-factor.

6. Compositions according to Claim 5, characterized in that the said supplementary elements are in concentrations such that they display the ratios $R_1$, $R_2$ ...... $R_n$ according to Claim 1.

7. Compositions according to Claim 5, characterized in that the said supplementary elements are in concentrations independent of the ratios $R_1$, $R_2$ ...... $R_n$ according to Claim 1.

8. Compositions according to any one of Claims 1 to 7, characterized in that the elements are in the form of pharmaceutically-acceptable and mutually-compatible salts.

9. Process for the preparation of compositions according to one of Claims 1 to 8, intended for administration to a given living species, characterized:
 − in that determination is made, for a specific biological medium of the said given living species, for at least the mineral elements $E_1$, $E_2$ ...... $E_n$ in the following finite numbers: Rb, K, Mg, Se and Zn, of the values of the physiological concentrations $Cx_1$, $Cx_2$ ...... $Cx_n$ of these elements in the said medium,
 − in that a determination is made of the values of the concentration ratios:

$$R_1 = \frac{Cx_1}{Cx_1} \quad R_2 = \frac{Cx_2}{Cx_1} \quad ...... \quad R_n = \frac{Cx_n}{Cx_1}$$

of the said elements $E_1$, $E_2$ ...... $E_n$, in the said medium, as well as the limit values of the said ratios,
 − in that a multi-element composition is prepared by mixing at least the said elements $E_1$, $E_2$ ...... $E_n$ in concentrations $C_1$, $C_2$ ...... $C_n$ such that the values of the concentration ratios of the said multi-element composition lie respectively between the limit values of the ratios $R_1$, $R_2$ ...... $R_n$,
 − in that the said composition is placed in a form that can be assimilated by the said species.

10. Use of the compositions according to any one of Claims 1 to 8, to obtain a drug designed to favour the supplementing activity of the supplementary element(s) added to the basic multi-element composition.

11. Use of the compositions according to any one of Claims 1 to 8, to obtain a drug designed to activate the restoration of equilibrium of the said supplementary element(s) added to the basic multi-element composition.

12. Use of the compositions according to any one of Claims 1 to 8, to obtain a drug designed to stimulate the metabolism of the adipose cells.


## Claims for the following Contracting States : ES, GR

1. Process for the preparation of compositions of mineral elements, designed hereinafter as multi-element compositions, intended for administration to a given living species, characterized:

– in that determination is made, for a specific biological medium of the said given living species, for at least the mineral element $E_1$, $E_2$ ...... $E_n$ in the following finite numbers: Rb, K, Mg, Se and Zn, of the values of the physiological concentrations $Cx_1$, $Cx_2$ ...... $Cx_n$ of these elements in the said medium,

– in that a determination is made of the values of the concentration ratios:

$$R_1 = \frac{Cx_1}{Cx_1} \quad R_2 = \frac{Cx_2}{Cx_1} \quad ...... \quad R_n = \frac{Cx_n}{Cx_1}$$

of the said elements $E_1$, $E_2$ ...... $E_n$, in the said medium, as well as the limit values of the said ratios,

– in that a multi-element composition is prepared by mixing at least the said elements $E_1$, $E_2$ ...... $E_n$ in concentrations $C_1$, $C_2$ ...... $C_n$ such that the values of the concentration ratios of the said multi-element composition lie respectively between the limit values of the ratios $R_1$, $R_2$ ...... $R_n$,

– in that the said composition is placed in a form that can be assimilated by the said species.

2. Process according to Claim 1, characterized in that the basic composition contains at least one other supplementary mineral element.

3. Process according to Claim 2, characterized in that the said supplementary elements are in concentrations according to Claim 1.

4. Process according to Claim 2, characterized in that the said supplementary elements are in concentrations independent of the ratios $R_1$, $R_2$...... $R_n$ according to Claim 1.

5. Process according to Claim 1, characterized in that the basic composition contains at least one other supplementarry mineral element and/or a co-factor.

6. Process according to Claim 5, characterized in that the said supplementary elements are in concentrations such that they display the ratios $R_1$, $R_2$ ...... $R_n$ according to Claim 1.

7. Process according to Claim 5, characterized in that the said supplementary elements are in concentrations independent of the ratios $R_1$, $R_2$ ...... $R_n$ according to Claim 1.

8. Process according to any one of Claims 1 to 7, characterized in that the elements are in the form of pharmaceutically-acceptable and mutually-compatible salts.

9. Use of the compositions according to any one of Claims 1 to 8, to obtain a drug designed to favour the supplementing activity of the supplementary element(s) added to the basic multi-element composition.

10. Use of the compositions according to any one of Claims 1 to 8, to obtain a drug designed to activate the restoration of equilibrium of the said supplementary element(s) added to the basic multi-element composition.

11. Use of the compositions according to any one of Claims 1 to 8, to obtain a drug designed to stimulate the metabolism of the adipose cells.